# EUROPEAN PATENT APPLICATION

(11) **EP 4 287 193 A1**
(43) Date of publication of application: **06.12.2023**
(21) Application number: 22176227.1
(22) Date of filing: 30.05.2022
(51) Int. Cl.: G16H 10/40

(54) **COMPUTER-IMPLEMENTED METHODS AND ASSESSING FOR THE PRESENCE OF AN ANOMALY IN A LABORATORY INSTRUMENT**

(71) Applicant: Beckman Coulter, Inc., Brea, CA 92821 (US)
(72) Inventor: MACKOWIAK, Stephan, 81249 Munich (DE); LINKE, Marco, 80339 Munich (DE)
(74) Representative: Müller-Boré & Partner Patentanwälte PartG mbB

(57) **Abstract**

An aspect of the present invention relates to a computer-implemented method comprising the steps of: obtaining, from or by a first computing device, log data, the log data comprising a first plurality of log data items; and obtaining anomaly data, the anomaly data comprising information indicative of the presence of an anomaly in a laboratory instrument, wherein the first computing device is configured to access a log file of the laboratory instrument, the log file comprising a first plurality of log file entries, each log file entry of the first plurality of log file entries comprising a plurality of fields, and to construct the log data, by, for each log data item of the first plurality of log data items: selecting first field-data of a first field of a respective log file entry, the first field of the respective log file entry specifying a respective event which has occurred in the laboratory instrument; selecting second field-data of a second field of the respective log file entry, the second field of the respective log file entry further describing the respective event occurred in the laboratory instrument, wherein the selecting of the respective second field is based on the first field-data of the respective first field of the respective log file entry; and constructing said each log data item by using the first field-data of the first field of the respective log file entry and the second field-data of the second field of the respective log file entry. Additional aspects of the present invention relate to further computer-implemented methods, a data processing system, a laboratory instrument, an automated laboratory system, a computer program product, and a computer-readable medium.

## Description

### Technical field

The present invention relates to analysing and/or testing biological samples.

### Background

The reliability of results from investigating biological samples (herein also referred to as "sample") in an automated laboratory system (herein also referred to as "ALS") depends on whether the laboratory instruments comprised by the ALS perform the tasks associated to them correctly and completely. In case of an error in the laboratory instruments, which might deteriorate the reliability of results, it is difficult to detect such an error or anomaly after the investigation of a biological sample has been completed due to the huge number of samples investigated by an ALS as well as the number and the complexity of the laboratory instruments.

### Summary

It is therefore an object of the present invention to render analysing and/or testing of a biological sample more reliable. Specifically, improved computer-implemented methods are provided.

This object is solved by the appended independent claims. Specific embodiments result from the respective dependent claims.

A first aspect of the present invention relates to a computer-implemented method comprising the steps of:
- obtaining, from or by a first computing device, log data, the log data comprising a first plurality of log data items; and
- obtaining anomaly data, the anomaly data comprising information indicative of the presence of an anomaly in a laboratory instrument,
wherein the first computing device is configured to access a log file of the laboratory instrument, the log file comprising a first plurality of log file entries, each log file entry of the first plurality of log file entries comprising a plurality of fields, and to construct the log data, by, for each log data item of the first plurality of log data items:
o selecting first field-data of a first field of a respective log file entry, the first field of the respective log file entry specifying a respective event which has occurred in the laboratory instrument;
o selecting second field-data of a second field of the respective log file entry, the second field of the respective log file entry further describing the respective event which has occurred in the laboratory instrument, wherein the selecting of the respective second field is based on the first field-data of the respective first field of the respective log file entry; and
o constructing said each log data item by using the first field-data of the first field of the respective log file entry and the second field-data of the second field of the respective log file entry.

In particular, the presence of the anomaly is assessed by using the log data. Herein, the respective event may also be referred to as the event associated to the respective log data item and/or as the event associated to the respective log data entry.

Advantageously, according to the aforementioned first aspect of the invention, the selection of the first and second field allows for simplifying the data by dropping irrelevant information and for making manifest the correlation between the log file entries of the log file. In other words, it allows better data correlation by constructing the log data items from or mapping the log data items with the log file entries. Thus, the method according to the first aspect of the invention allows for a better, faster and/or easier processing of data. Alternatively or additionally, the log data are used for detecting anomalies and the improved data correlation among the log data items leads to a better, faster and/or easier detection of anomalies. Hence, the reliability of the results of sample analyses provided by, for example, laboratory instruments of an automated laboratory system is increased.

According to the present invention, a computing device, e.g. the first computing device mentioned above, may comprise or be a cloud computing system or platform. It may comprise or be a distributed computer system. A computing device may comprise or be a network of connected desktop PCs and/or workstations and/or laptops and/or tablet PCs or the like. The network may be a LAN and/or a wireless LAN. In particular, according to the present invention, a computing device may be a desktop PC, a workstation, a laptop or a tablet PC or the like. As described herein, a computing device, e.g. the first computing device, may comprise one or more processors, e.g. a CPU, a GPU, a Tensor Processing Unit (TPU), or the like and a memory.

The first computing device may be comprised in the laboratory instrument. Alternatively, the first computing device may not be comprised in the laboratory instrument or in an ALS comprising the laboratory instrument. In this case, in particular, the first computing device may be in data communication with the laboratory instrument. The first computing device may in particular be a server or a cloud computing system in data communication with the laboratory instrument, e.g. with a computing device thereof.

In particular, obtaining, by the first computing device, data, e.g. log data may comprise constructing the data, i.e. creating the data based on one or more inputs. For example, obtaining, by the first computing device, log data may comprise constructing, by the first computing device, the log data by using the first plurality of the log file. In yet another example, obtaining, by the first computing device, data, e.g. log data may comprise accessing a first portion of the data and generating a second portion of the data e.g. from the first portion of the data.

In some embodiments, the step of obtaining, from the first computing device, data, e.g. log data, may comprise accessing, e.g. by a second computing device, the data. In particular, said data may be created and/or provided to the first computing device by the second computing device. For example, in said embodiments the first computing device is comprised in the laboratory instrument or in an ALS comprising the laboratory instrument. Moreover, in said embodiments, the second computing device may be a server or a cloud computing system configured to (i) assess, by using the log data, for the presence of the anomaly in the laboratory instrument and (ii) generate the anomaly data. In particular, the second computing device is different from the first computing device.

In the present disclosure, "accessing, by a computing device (e.g. the second computing device), data" may comprise retrieving the data e.g. from the at least one memory of said computing device, from the memory of another computing device (e.g. the first computing device), or from another remote data storage (a database, a secondary memory, a cloud storage or the like). Accordingly, in some cases, retrieving data may comprise downloading data. Exemplarily, said computing device may retrieve data in response to a user command and/or to a notification sent by another computing device, e.g. the first computing device. Additionally or alternatively, "accessing, by a computing device, data" may comprise receiving the data, e.g. from a user or another computing device. These options are not mutually exclusive. For instance, accessing, by a computing device, data may comprise receiving the data, storing the data in the memory of said computing device and retrieving the data by accessing said memory.

According to the first aspect, the log file is a file comprising a plurality of log file entries. The log file may be created by a laboratory instrument or an automated laboratory system during its operation, e.g. before, after and/or while one or more samples are analyzed or processed by the laboratory instrument or the ALS. The log file may comprise one or more log file entries each of these log file entries comprising information that specifies a respective event that is occurred in the laboratory instrument. The respective event may refer to a respective sample tube or a respective component of the laboratory instrument. For example, the log file may contain log file entries which describe events referring to a sample tube and log file entries which describe events referring to another sample tube. Alternatively or additionally, the log file may contain log file entries which describe events referring to a component of the laboratory instrument and log file entries which describe events referring to another component of the laboratory instrument. Alternatively, the log file may comprise or consist of log file entries describing events pertaining to a single component of the laboratory instrument or to a single sample tube. Alternatively or additionally, the log file may be organized in a database.

In some embodiments, the log file entries may be comprised in JSON strings and/or the log file may be organized by using a mark-up language, e.g. HTML, latex or the like. In this case, each log file entry may be marked by a log file entry tag and each entry. Alternatively or additionally, the fields of the log file entries may be divided into categories and marked in the log file by a category-dependent field tag.

The log file may comprise a list of log file entries. The log file entries in the list maybe sorted chronologically according to the creation time of the log file entries, e.g. from the least recently generated entry to the most-recently generated entry or from the least recently generated entry to the most-recently generated entry. Alternatively or additionally, the log file entries in the list maybe sorted chronologically according to the occurrence time of the events described by the entries, e.g. from the entry describing the least recently occurred event to the entry describing the most-recently occurred event or from the entry describing the most recently occurred event to the entry describing the least recently occurred event.

For example, an event specified by a log entry may be a change of the state of a sample which is processed by the laboratory instrument. For instance, an event specified by a further log entry of the log file may be the event that a sample tube has reached or left a component of the laboratory instrument. In particular, an event specified by another log entry of the log file may be the event that a component of the laboratory instrument has started or finished the execution of instructions, e.g. to carry out one or more processing steps (for example an analysis on a sample). An event specified by a further log entry of the log file may be the approval or rejection of the route plan of a sample.

In particular, one or more log file entries of the log file comprise a plurality of fields. For example, a log file entry may comprise a field comprising data that specify an event which has occurred in the laboratory instrument. Moreover, a log file entry may comprise one or more fields comprising data that further describes the event which has occurred in the laboratory instrument. Furthermore, a log file entry may comprise a field comprising data that specify the timepoint at which the log file entry is created and/or a field comprising data that specify the timepoint at which an event which has occurred in the laboratory instrument. Furthermore, a log file entry may comprise a field comprising data that specify the sample to which the log file entry refers. For example, if the log file entry specifies a change of the state of a sample which is processed by the laboratory instrument, said log file entry comprises a field comprising data that uniquely determine the sample that has changed state.

In particular, each log file entry of the log file may be a list comprising one or more alphanumeric strings, each alphanumeric string constituting the field data a respective field of the log file entry. For example, the strings of the log file entry may be separated by a separator, e.g. a character such as a comma, a semicolon, a slash, or the like.

The first field data may comprise the title of the respective event or one or more identifiers of the respective event. The second field comprises second field-data which describes the respective event. The second field-data may comprise identifiers or descriptors of the processing step which the laboratory instrument performed or the location of the component of the laboratory instrument in which the respective event has occurred.

For example, if the respective event is the change of state of a sample, the first field data may comprise or consist of an alphanumeric string specifying that a change in the state of the sample has occurred, e.g. the string "SampleStateChanged". In this case, the second field data may comprise or consist of an alphanumeric string specifying the current state of the sample. For instance, if the sample has become ready for scheduling, the alphanumeric string of the second field data may be or comprise the string "ReadyForSchedulingState". Alternatively, if the processing of the sample has been completed, the alphanumeric string of the second field data may be or comprise the string "CompletedState".

For instance, if the respective event is the event that a sample tube has reached a component of the laboratory instrument, the first field data may comprise or consist of an alphanumeric string specifying that said sample tube has reached a component of the laboratory instrument, e.g. the string "SiteReached". In this case, the second field data may comprise or consist of an alphanumeric string specifying which component has been reached. For instance, if the sample has reached the storage, the alphanumeric string of the second field data may be or comprise the string "Storage". If, instead, the sample has reached the analyzer AU5800, the alphanumeric string of the second field data may be or comprise the alphanumeric string "AU5800".

In particular, the step of obtaining the anomaly data may be carried out by the first computing device.

Alternatively, the step of obtaining the anomaly data may be carried out by the second computing device. In particular, in this case, the anomaly data may be obtained from the first computing device.

In some embodiments, the step of obtaining, e.g. by the second computing device, anomaly data may comprise generating said data e.g. based on the log data. For example, obtaining, by the first computing device, log data may comprise constructing, by the first computing device, the log data by using the first plurality of the log file. In particular, in this case, the method may comprise the step of providing the anomaly data to the first computing device. In particular, in these embodiments, the step of obtaining the log data may comprise accessing said data and is carried by the second computing device.

In yet another example, the step of obtaining anomaly data may comprise accessing a first portion of the anomaly data and generating a second portion of the anomaly data e.g. from the first portion of said data.

In some embodiments, the step of obtaining anomaly data may comprise accessing said data. In particular, in these embodiments, said step may be carried out by the first computing device, which obtains the anomaly data from the second computing device. Additionally, in these embodiments, the second computing device may be configured to generate the anomaly data by using the log data.

The selecting of data of a field of a log file entry may comprise choosing or determining the data according to a specific pattern or rule. In particular, the selecting of data of a field of a log file entry may comprise selecting the field of the log file entry and accessing and, optionally, storing the data comprised in the field.

For example, for at least a log data item of the first plurality of the log data items the selecting of the first field-data of the first field of the respective log file entry comprises choosing as first field the field at a given position in the log file entry. For instance, the log file entries of the log file may consist of a list of fields and may be configured so that the field at the k^{th} position of the list comprises data specifying an event which has occurred in the laboratory instrument. In this case, for at least a log data item of the first plurality of log data items, the selecting of the first field-data of the first field of the respective log file entry comprises choosing the field at the k^{th} position of the list as the first field and accessing the data contained in the first field, the data contained in the first field being the first field data.

For example, for at least a log data item of the first plurality of the log data items the selecting of the second field-data of the second field of the respective log file entry comprises determining a respective position in the respective log file entry by using the first field data, choosing as second field the field at the respective position in the respective log file entry and accessing the data contained in the second field, the latter data being the second field data.

In particular, the respective position may be determined by using a first look-up table correlating the possible values of the first field data with the positions on the log file entries. For instance, if the first field-data of the first field of the respective log entry consists of the alphanumeric string "SampleStateChanged", the respective position may be determined by using the first look-up table to be the m₁^{th} position in the respective log entry. In this case, the field at the m₁^{th} position in the respective log file entry is chosen as second field. The data contained in the second field is the second field-data and, e.g. may be the alphanumeric string "ReadyForSchedulingState" or the string "CompletedState". If, instead, the first field-data of the first field of the respective log entry consists of the alphanumeric string "SiteReached", the respective position may be determined by using the first look-up table to be the m₂^{th} position in the respective log entry. In this case, the field at the m₂^{th} position in the respective log file entry is chosen as second field. In this case, the data contained in the second field is the second field-data and, e.g. may be the string "Storage" or the alphanumeric string "AU5800" .

Alternatively, if the log file is organized by a using mark-up language, the selecting of the second entry may be carried out by selecting, based on the first entry, the appropriate tag among the tags which marks the entries of the log file entry.

According to the first aspect of the invention, the log data comprises a plurality of log data items, L₁, L₂, ..., L_{N-1}. In particular, for each i = 1, 2, ..., N-1, the log data item Lᵢ refers to a respective event, Eᵢ, occurred in the laboratory instrument. For example, the respective event Eᵢ is the event described by the respective log entry used to construct the log data item Lᵢ. The log data may comprise N-1 strings, wherein N may be an integer greater than or equal to 1. N may equal to 1, 2, 3, 4, 5, 6, 7, 8, etc. Each string may correspond to a log data item comprised by the log data. The log data may be used to detect an anomaly in a laboratory instrument.

For instance, the log data may be a list comprising the plurality of log data items. In particular, each log data item may be an alphanumeric string constructed by using the first field-data of the first entry of the respective log entry and the second field-data of the second entry of the respective log entry. The log data may be stored in one or more files. Additionally or alternatively, the log data may be stored in a memory of the first computing device and/or in a memory of the second computing device. The log data item comprised in the log data may refer to a single sample tube or to the same component of the laboratory instrument.

The log data items in the log data may be sorted in the log data according to the order in which the respective log file entries are sorted in the log file. In particular, the log data items in the log data are sorted chronologically, so that a log data item precedes another log data item in the log data if the event related to the former log data item entry occurred before the event related to the latter log data item or so that a log data item precedes another log data item in the log data if the event related to the former log data item entry occurred after the event related to the latter log data item. Alternatively or additionally, the log data items in the list may be sorted chronologically, so that a log data item precedes another log data item in the log data if the respective log file entry associated to the former data item has been generated before the respective log file entry associated to the latter data item or so that a log data item precedes another log data item in the log data if the respective log file entry associated to the former data item has been generated after the respective log file entry associated to the latter data item.

The log data items may be constructed by using N-1 respective log file entries. The N-1 respective log file entries consist of a first log file entry, which refers to a respective sample tube or to a respective laboratory instrument component, and the first N-2 log file entries that follow the first log file entry in the log file and, respectively, refer to said respective sample tube or said respective laboratory instrument component, respectively.

The constructing of a log data may comprise correlating the log file entries and the log data items and/or mapping the plurality of log file entries onto the plurality of log data items according to a specific map.

In particular, a log data item may be constructed by using a second look-up table correlating the possible values of the log data items with the possible values of the first field-data and of the second field-data comprised in the respective log file entry. For example, if the first field-data of the first field and the second field-data of the second field of the respective log entry consist of the alphanumeric strings "SiteReached" and "Storage", respectively, the log data item may be the string "StorageReached".

In some embodiments, a log data item may be constructed by concatenating the first field-data of the first field of the respective log entry and the second field-data of the second field of the respective log entry. Said concatenation may be carried out by a separator, e.g. a dot, a comma, a slash or the like. For instance, if the first field-data of the first field and the second field-data of the second field of the respective log entry consist of the alphanumeric strings "SiteReached" and "Storage", respectively, the log data item may be the string "SiteReached.Storage".

In particular, the possible values of the log data items, e.g. the possible alphanumeric strings that may be constructed from log files, constitute a log data vocabulary with a finite number of lemmas, each lemma of said log data vocabulary being exactly one of the possible values of the log data items. For example, the log data vocabulary comprises the lemmas "SiteReached.AU5800", "SiteReached.Storage", "SampleStateChanged.ReadyForSchedulingState" and "SampleStateChanged .CompletedState".

According to the first aspect of the invention, the anomaly data comprises information indicative of the presence of an anomaly in a laboratory instrument, i.e. whether or not an anomaly occurred in the laboratory instrument. Using the anomaly data, an anomaly in a laboratory instrument may be detected. The anomaly data may be generated from or based on the log data.

For example, the anomaly data may comprise or consist of an alphanumeric string specifying that an anomaly has occurred, such as the alphanumeric string "Anomaly occurred". Additionally or alternatively, the anomaly data may comprise or consist of a numerical value between 0 and 1, said value providing an estimate of the probability that an anomaly has occurred. Additionally or alternatively, the anomaly data may comprise or consist of an alphanumeric string which uniquely specifies that type of anomaly that has occurred. The anomaly data may also comprise information specifying how the anomaly may be resolved.

The anomaly data may be displayed to a user or sent to a user as a text and/or email message and/or be printed by a user, in particular to provide guidance how to resolve the anomaly in the laboratory instrument.

An anomaly may be a malfunction and/or a failure of at least a component of the laboratory instrument which may prevent the laboratory instrument from performing its tasks and/or analyses on a sample correctly. This malfunctioning and/or failure may be originated by defective parts of the laboratory instrument.. The anomaly may comprise the situation where, for example, the motor of a centrifuge may not be working properly so that the sample may not be centrifuged completely. Moreover, the anomaly may be originated by an nsufficient amount of reagent loaded in a component of the laboratory instrument.

Alternatively or in addition, the anomaly may be originated by defects of a sample tube, such as a misplaced label, the presence of a contaminant in the sample, an insufficient amount of sample in the tube, or the like. An anomaly may also be a deterioration of the performance of a component of the lab instrument e.g. caused by an overload of the component and/or of the instrument. For instance, said deterioration may be an increase of the average processing time that the component takes to carry out the processing step it is configured to carry out. An anomaly may also be interruption of the processing or the sample e.g. caused by an unexpected behaviors of a component of the instrument. For instance, an unexpected behavior may be an unexpected movement of a robotic arm of the instrument.

The lab instrument may comprise or consist of one or more standalone instruments. The lab instrument may also comprise or consist of an automated laboratory system. The laboratory instrument may comprise a robotic aiding device, such as a robotic arm, and/or conveying means to convey a sample and/or transfer the sample from an instrument to another instrument, e.g. from a standalone instrument to an automated lab system.

In an embodiment of the invention, a lab instrument may comprise or consist of a pre-analytical instrument, and/or a post-analytical instrument and/or an analytical instrument. The laboratory instrument may for example be a centrifuge.

Generally, an automated laboratory system (hereinafter also referred to as: "ALS") is an assembly comprising a plurality of components and a computing device, wherein the computing device is operatively connected to these components and is configured to control each component. A component may be an analytic instrument, a pre-analytic instrument, a post-analytic instrument, an input/output module, a transportation component (e.g. track, belt, tube carrier) configured to move a sample or the like.

An analytic instrument (hereinafter also referred to as "analyser") is an instrument configured to carry out one or more analytical steps, such as measuring one or more characteristics of the sample, e.g. the concentration of a given analyte. A pre-analytic instrument is an instrument configured to carry out one or more pre-analytic steps on a biological sample to prepare said sample for the analytic instrument(s). For example, centrifuges, aliquoters and de-cappers are pre-analytic instruments. A post-analytic instrument is an instrument configured to carry out one or more post-analytic steps on the biological sample after the sample has been processed by one or more analytic instruments. For example, re-cappers and sample storage units are post-analytic instruments.

The laboratory instrument may be comprised by an automated laboratory system. It may be part of or comprised by a laboratory or a laboratory complex or a laboratory facility. The laboratory instrument may be programmable.

In an embodiment, the step of obtaining the anomaly data may comprise assessing, by using the log data, for the presence of the anomaly in the laboratory instrument and generating the anomaly data.

The anomaly data may be generated based on the log data. The generation may comprise removing or discarding log data items which are not relevant for anomaly detection, specifically which do not contain information about an anomaly. The assessment for the presence of an anomaly by using the log data may be done by inspecting and/or evaluating the log data for information indicating the presence of an anomaly.

In an embodiment, the log data may be obtained from the first computing device and the step of obtaining the anomaly data may be carried out by the second computing device.

In particular, in this embodiment, the step of obtaining the anomaly data may comprise assessing, by using the log data, for the presence of the anomaly in the laboratory instrument and generating the anomaly data. Moreover, in this embodiment, the log data are obtained, e.g. by the second computing device, from the first computing device. In particular, this embodiment of the method according to the first aspect is carried out by the second computing device. In this case the first computing device may be comprised in the laboratory data and the second computing device is a server or a cloud computing system in data communication with the first computing device. For example, the second computing device may obtain, e.g. receive, the log data from the first computing device and obtain the anomaly data by assessing, by using the log data, for the presence of the anomaly in the laboratory instrument.

In an embodiment, the assessing for the presence of an anomaly in the laboratory instrument may be carried out by using the log data as input to at least one of a machine learning algorithm and/or a decision tree. For example, the anomaly data may be an output of the machine learning algorithm. In particular, the machine learning algorithm is a trained machine learning algorithm.

Advantageously, the application of a machine learning algorithm or a decision tree for identifying an anomaly in a laboratory instrument makes anomaly detection more precise, and thus allows for more reliable results regarding testing and/or analyzing a sample.

The machine learning algorithm may be configured to initiate the resolution of the anomaly by generating, based on the anomaly data, resolution data, and by providing said instructions to the laboratory instrument, e.g. to the first computing device, if the first computing is comprised in the laboratory instrument. In particular, the resolution data comprise information indicative of one or more actions for resolving the anomaly present in the laboratory instrument. For instance, the resolution data may comprise instructions for reconfiguring and/or re-programming the laboratory instrument and/or for re-routing one or more sample, so that to resolve or avoid the anomaly.

In an embodiment, the machine learning algorithm may comprise or consist of a neural network. The use of a neural network, improves the reliability of anomaly detection, as it is trained on non-anomalous data and, hence, allows for detecting also unforeseen and/or rare anomalous behaviors which were not known and/or observed during the training of the machine learning algorithm.

In particular, the neural network may comprise at least one of: a convolutional neural network, a recursive neural network, a recurrent neural network, a long short-term memory recurrent neural network, a sequence-to-sequence model, or a shallow neural network.

These types of neural networks render the anomaly detection particularly accurate. Thus, the reliability of results regarding sample testing and/or analyzing may be improved further.

In particular, the neural network may comprise at least a layer comprising a plurality of long short-term memory units. Alternatively or additionally, the neural network may comprise a dense layer as output layer. The dense layer may comprise a plurality of nodes, each node comprising an activation function such as a sigmoid function, a hyperbolic tangent, a ReLu function, and the like.

In an embodiment, the method may comprise the step of:
- obtaining, by or from the first computing device, a benchmark log data item, L_{N}, by using a respective benchmark log file entry of the log file,
wherein the assessing for the presence of an anomaly in the laboratory instrument is based on the benchmark log data item.

In particular, the benchmark log data item may be so that an event related to the benchmark log data item occurred after the events related to the log data items of the log data. Alternatively or additionally, the benchmark log data item may be so that the benchmark log file entry has been generated after the respective log file entries used to construct the log data items of the log data. For example, the benchmark log data item may be compared with the output of the neural network to assess for the presence of the anomaly.

In an embodiment, the step of obtaining, by the first computing device, the benchmark log data item may comprise:
- selecting first benchmark-field-data of a first field of the respective benchmark log file entry, the first field of the benchmark log file entry specifying a respective event which has occurred in the laboratory instrument;
- selecting second benchmark-field-data of a second field of the respective benchmark log file entry, the second field of the benchmark log file entry further describing the respective event which has occurred in the laboratory instrument, wherein the selecting of the second field of the benchmark log file entry is based on the first benchmark-field-data of the first field of the benchmark log file entry; and
- constructing said benchmark log data item by using the first benchmark-field-data of the first field of the respective benchmark log file entry and the second benchmark-field-data of the second field of the respective benchmark log file entry.

The benchmark fields and benchmark field-data may be specified as described above for the fields and field-data of the log file entries of the log file.

For instance, the respective benchmark log file entry is the first log file entry of the log file that follows the N-1 respective log file entries used to construct the log data items of the log data and refer to the sample tube or the laboratory instrument component, to which the log data items of the log data refer. In particular, the benchmark log data item is related to the least recent event that (i) follows the events related to the log data items of the log data and (ii) refers to same sample tube or the laboratory instrument component to which the log data items of the log data refer. Hence, in absence of anomalies of the instrument, the possible values of the benchmark log data item are correlated with the log data items L₁, L₂, ..., L_{N-1}.

Exemplarily, the step of obtaining, by the first computing device, the benchmark log data item may comprise selecting the benchmark log file entry. In particular, selecting the benchmark log file entry comprises determining the first log file entry of the log file that follow the N-1 respective log file entries used to construct the log data items of the log data and refer to the sample tube or the laboratory instrument component, to which the log data items of the log data refer.

In an embodiment, the assessing, by using the log data, for the presence of the anomaly in the laboratory instrument may comprise:
- generating output data by using the log data, the output data comprising information indicative of one or more expected benchmark log data items, and
- assessing for the presence of the anomaly in the laboratory instrument by using the output data and the benchmark log data item.

In some embodiments, the output data is the output of the neural network.

For example, each expected benchmark log data item of the one or more expected benchmark log data items refers to an event that, in absence of anomalies, (i) refers to the same sample tube or laboratory instrument component to which the log data items of the log data refer, and, in absence of anomalies, (ii) could immediately follow the events related to the log data items of the log data. In this case, each expected benchmark log data item of the one or more expected benchmark log data items is one of the log data items that, in absence of anomalies, is expected to immediately follow the log data items of the log data.

According to the present invention, an event E_{A} referring to the same sample tube or laboratory instrument component, to which the events, E₁, E₂, ..., E_{N-1} refer, immediately follows the events E₁, E₂, ..., E_{N-1} if no other events referring to said sample tube or laboratory instrument component occurred before the event E_{A} and after each of the events E₁, E₂, ..., E_{N-1}. Moreover, a log data item L_{F} related to an event E_{F} and referring to the same sample tube or laboratory instrument component, to which the log data items, L₁, L₂, ..., L_{N-1} refer, immediately follows the log data items L₁, L₂, ..., L_{N-1} if the event E_{F} immediately follows the events E₁, E₂, ..., E_{N-1}.

In particular, the one or more expected benchmark data constitute a subset, e.g. a proper subset, of the log data vocabulary. In the following, said subset may be also referred to as "benchmark subset".

For example, the benchmark subset may comprise the S lemma of the log data vocabulary that are most likely to immediately follow the log data items L₁, L₂, ..., L_{N-1}. In this case, in particular, the assessing for the presence of the anomaly comprises determining whether the benchmark log data item L_{N} is comprised in the benchmark subset. The anomaly is considered present, and the anomaly data are generated, if the benchmark subset does not comprise the log data item L_{N}.

In other words, according to the above-mentioned embodiment, the log data items L₁, L₂, ..., L_{N-1} may be processed to generate an output ("output data"), which may specify one or more predictions of the L_{N} string based on L₁, L₂, ..., L_{N-1}. The output data and L_{N} may be used, e.g. compared to detect the anomaly. For example, it may be checked if the output data and the log data item L_{N} are compatible with one another. In an embodiment, the output data may comprise information indicative of a probability for the benchmark log data item to be equal to the one or more expected benchmark log data items.

In particular, the output data may comprise information specifying the benchmark subset and information specifying, for each lemma of the benchmark subset the probability that, in absence of an anomaly, said each lemma would immediately follow the log data items L₁, L₂, ..., L_{N-1}. In this case, in particular, the assessing for the presence of the anomaly comprises determining whether the benchmark log data item L_{N} is comprised in the benchmark subset and/or whether the probability associated to L_{N} is greater than or equal to a given threshold or is among the T lemmas with the highest probability.

In other words, according to this embodiment, the output data may represent the probability that the N^{th} string of the log data is equal to the prediction of the N^{th} string based on the N-1 strings of the log data. For example, an anomaly may be detected if this probability is lower than a predetermined threshold.

In an embodiment, if it has been assessed that the anomaly is present in the laboratory instrument, the method may further comprise the step of obtaining resolution data, the resolution data comprising information indicative of one or more actions for resolving the anomaly present in the laboratory instrument.

The resolution data may comprise one or more suggestions as to how to resolve the anomaly. For example, the anomaly detected may be a robotic arm moving a sample too fast and/or pushing a sample too hard. Thus, the resolution data may for example instruct an operator to check if the robotic arm is working as expected . Specifically, it may include the suggestion of readjusting for example a joint of the robotic arm. In particular, the anomaly data may comprise, e.g. consist of, resolution data.

The step of obtaining the anomaly data may be carried out by the second computing device. In particular, in this case, the resolution data may be obtained from the first computing device e.g. by receiving or retrieving said data.

In some embodiments, the step of obtaining resolution data may comprise accessing said data. In particular, in these embodiments, said step may be carried out by the first computing device, which obtains the resolution data from the second computing device. Additionally, in these embodiments, the second computing device may be configured to generate the resolution data by using the log data and/or the anomaly data.

The resolution data may be printed and/or displayed to a user, suggesting to the user how to resolve the anomaly, in particular which actions to take to resolve the anomaly. This way, the user is guided in properly operating and/or properly maintaining the laboratory instrument. The resolution data may also be used by a machine learning algorithm to resolve the anomaly detected by the machine learning algorithm, e.g. by modifying the route plan of one or more sample tubes to bypass the laboratory instrument in which the anomaly was detected. In this way, an automated laboratory system to autonomously test and/or analyze a sample and detect and/or correct anomalies such as errors in its laboratory instrument or laboratory instruments.

In an embodiment, the step of obtaining resolution data may comprise generating, by using the log data, the resolution data and, optionally, providing resolution data to the first computing device. In particular, in this case, the step of obtaining resolution data may be carried out by the second computing device.

The resolution data may be generated by applying a machine learning algorithm, in particular a neural network, as specified above, or a decision tree to the log data. The resolution data may be printed and/or displayed via the first computing device.

In another embodiment, the output of the machine learning algorithm may comprise or be an error code. In particular, the training of the machine learning algorithm may be carried out by using anomalous and non-anomalous log data items and the output of the trained machine learning algorithm may be unambiguously related to an error code specifying the presence or absence of an anomaly, and if an anomaly is present, the type of anomaly. The diagnosis for resolving an anomaly being detected may be made by a look-up table correlating the error code to possible solutions.

Herein, providing data to a computing device may be carried out by making said data available to said computing device, e.g. by storing said data in a memory comprised in or accessible by said computing device and/or by granting said computing device access to said data, so that said computing device can retrieve, copy and/or access said data. For example, providing data to a computing device may comprise sending, e.g. over a network, said data to said computing device. Providing data to a computing device may comprise sending, e.g. over a network, to said computing device a link to said data..

In an embodiment, the step of obtaining, by the first computing device, log data may comprise:
- accessing the log file of the laboratory instrument; and
- constructing the log data,
wherein the step of constructing the log data comprises, for each log data item of the first plurality of log data items:
o selecting the first field-data of the first field of the respective log file entry;
o selecting the second field-data of the second field of the respective log file entry, wherein the selecting of the second field is based on the first field-data of the first field of the respective log file entry; and
o constructing said each log data item by using the first field-data of the first field of the respective log file entry and the second field-data of the second field of the respective log file entry.

The log file may be accessed at a shared site, such as a network storage, in particular a cloud storage. In particular, the log file may be accessed at a memory comprised in the laboratory instrument. For example, if the first computing device is comprised in the laboratory instrument, the log file may be stored in and accessed at the memory of the first computing device.

In an embodiment, the method according to the first aspect may further comprise the step of:
- providing, by the first computing device, the log data to a third computing device, the third computing device being configured to assess, by using the log data, for the presence of the anomaly in the laboratory instrument,
and wherein the step of obtaining, by the first computing device, log data may comprise:
- accessing the log file of the laboratory instrument; and
- constructing the log data, wherein the step of constructing the log data comprises, for each log data item of the first plurality of log data items:
   o selecting the first field-data of the first field of the respective log file entry;
   o selecting the second field-data of the second field of the respective log file entry, wherein the selecting of the second field is based on the first field-data of the first field of the respective log file entry; and
   o constructing said each log data item by using the first field-data of the first field of the respective log file entry and the second field-data of the second field of the respective log file entry.

According to this embodiment, in particular, the first computing device constructs the log data by selecting relevant information, thereby making manifest the correlation between the log file entries of the log file. Moreover, by distributing the method of the first aspect over a first computing device and a third computing device, which may be different from each other, the anomaly detection may be performed in a computerresource saving way, in particular memory-efficient way. As a result, the reliability of sample test and/or analysis results may be increased in an efficient manner with regards to use of computer hardware.

In particular, the third computing device may be the second computing device described herein. In particular, in this embodiment, the step of obtaining anomaly data may be carried out by the first computing device. For example, the anomaly data may be obtained from the third computing device, e.g. the second computing device. Additionally, the step of obtaining, by the first computing device, anomaly data may comprise receiving said data e.g. from the third computing device. The step of obtaining, by the first computing device, anomaly data may comprise retrieving said data from and/or accessing said data at a memory of the third computing device, e.g. of the second computing device.

In particular, in this embodiment the step of obtaining the anomaly data may comprise assessing, by using the log data, for the presence of the anomaly in the laboratory instrument and generating the anomaly data. Moreover, in this embodiment, the log data are obtained, e.g. by the second computing device, from the first computing device.

In particular, this embodiment of the method according to the first aspect is carried out by the first computing device. In this case, the first computing device may be comprised in the laboratory instrument and the second computing device is a server or a cloud computing system in data communication with the first computing device. In particular, the first computing device may construct the log data and provide said data to the second computing device, which is configured to assess for the presence of the anomaly. If the anomaly is present, the first computing device obtains, e.g. receives and/or retrieves, the anomaly data from the second computing device, which has generated said data.

In an embodiment, the constructing the log data may comprise, for each log data item of the first plurality of log data items,
- selecting time data of a respective third field of the respective log file entry, the time data comprising information indicative of the time, at which the respective log file entry has been created, and/or of the time, at which the respective event described by the first and/or second field of the respective log file entry has taken place;
wherein the constructing of said each log data item may be carried out by using the time data of the respective third field of the respective log file entry.

Advantageously, including the time data in constructing the log data items, which are used to assess for an anomaly in a laboratory instrument, allows for keeping track of the duration of the processing steps and, for detecting anomalies that cause delays in the completion of one or more processing steps. This way anomalies may be detected more precisely and/or a broader range of anomalies may be detected.

The time data may comprise at least one of time stamps, system time of the computing device of the laboratory analyzer and/or of the computing device gathering the log file entries, points in time in hours and/or minutes and/or seconds, time intervals, days, months, years or dates.

The third field and the respective field-data may be specified as described above for the first and second fields and their respective field-data.

In this embodiment, the method takes also into account the time of log file entries. In particular, the log data, which may be the data to be fed to a machine learning algorithm, may comprise N-1 strings and N-1 corresponding times, so that each string and each corresponding time may be obtained from a corresponding log file entry of the log file. In this case, the string entries and the corresponding times may be together in the same input.

In particular, by using relative time data, wherein the relative time data may be generated by using the time data and may comprise information indicative of the relative time at which the respective log file entry has been created, and/or of the time, at which the respective event described by the respective log file entry has taken place. The relative time may refer to a given time, e.g. with respect to the log file entries with earliest time entry.

In particular, in this embodiment, the log data comprises or consists of a list having a plurality of entries, D₁, D₂, ..., D_{N-1}. For each i=1, 2, ..., N-1, the entry Di is in turn a sublist comprising the log data item Li and the relative time value ΔTᵢ. In particular, for each i=1, 2, ..., N-1, ΔTᵢ = Tᵢ - T_{R}, wherein Tᵢ is the timepoint at which the event, Eᵢ, occurred in the laboratory instrument or at which the log entry associated to Ei was generated. The time point T_{R} is a reference time point which, for instance may be one of the timepoints T₁, T₂, ..., T_{N-1}, e.g. the smallest thereof.

Similarly, other "parameters" (i.e. numerical values) of the log file entries may be taken into account. For example, if the lab instrument comprises a robot, the value can be the current of the robot, possibly connected with other parameters present in the log file entry.

In an embodiment, the log data may comprise a second plurality of log data items, and the constructing the log data may comprise, for each log data item of the second plurality of log data items,
- selecting time data of a respective third field of the respective log file entry, the time data comprising information indicative of the time, at which the respective log file entry has been created, and/or of the time, at which the respective event described by the first and/or second field of the respective log file entry has taken place;
- constructing a respective log data item of the second plurality of log data items by using the time data of the respective third field of the respective log file entry.

Similarly to the previous embodiment, this embodiment allows for a more precise anomaly detection and/or for detecting a broader range of anomalies, e.g. the ones causing delays in the completion of one or more processing steps.

In the currently described embodiment, the method takes also into account the time of log entries. In particular, the log data, which may be fed to a machine learning algorithm, in particular a neural network, may comprise N-1 strings and N-1 corresponding times, so that each string and each corresponding time may be obtained from a corresponding log file entry of the log file. In this case, the string entries and the corresponding times may be together in the same input.

In particular, by using relative time data, wherein the relative time data may be generated by using the time data and comprise information indicative of the relative time at which the respective log entry has been created, and/or of the time, at which the respective event described by the respective log file entry has taken place. The relative time may be with respect to a given time, e.g. with the log file entries with earliest time entry.

In particular, in this embodiment, the log data may comprise a first list comprising the log data items L₁, L₂, ..., L_{N-1} and a second list comprising the aforementioned plurality of values ΔT₁, ΔT₂, ..., ΔT_{N-1}. For example, the first list may be processed by the machine learning algorithm, described above, e.g. a long short-term memory recurrent neural network, which processes the first list to generate the output data.

The second list may be processed by a further machine learning algorithm, e.g. a feed forward neural network. The latter algorithm may be a binary classifier trained to classify its input, e.g. the second list, as anomalous or non-anomalous. In particular, the further machine learning algorithm may be trained to classify as anomalous its inputs if one or more of the relative time values ΔT₁, ΔT₂, ..., ΔT_{N-1} lie outside a respective range.

In some embodiments, the first and the second list may be processed by the same neural network. For instance, the two lists may be combined in a single input for said neural network or cast into two different layers.

In an embodiment, each log data item of the first plurality of log data items may be associated with a single log file entry of the plurality of log file entries and each log data item of the second plurality of log data items may be associated with a single log file entry of the plurality of log file entries.

The log data items may be associated to the log file entries by a specific pattern and/or rule and/or map.

In an embodiment, each log file entry of the first plurality of log file entries may refer to a respective sample tube. Additionally or alternatively, for each log data item of the first plurality of log data items, the respective log file entry may refer to a given sample tube.

Herein, a sample tube may be a container for holding biological samples such as bodily fluids including blood, saliva, urine, etc.

A biological sample may be a sample of a bodily fluid of a subject. For example, the bodily fluid may be a physiological fluid, such as blood, saliva, urine, sweat, amniotic fluid, cerebrospinal fluid, ascites fluid, or the like. The biological sample may comprise one or more components that may potentially comprise analytes of interest for performing a clinical test on the sample. A clinical test may comprise one or more procedures that, when carried out on the biological sample or on a component thereof, allow for estimating the value of a parameter, e.g. a clinical parameter. In particular, a clinical test may comprise a physical, a biological, an optical, a mechanical, immunological, and/or chemical procedure.

The sample tube may be a cylindrical or cuboid container holding one or more of the aforementioned substances. The sample tube may be a cuvette. The sample tube may be closable and/or sealable. The sample tube may comprise a lid to close and/or seal the container holding one or more of the aforementioned substances. The sample tube may be configured to be positioned at or held by a laboratory instrument such as a centrifuge for example. The sample tube may be placed in a sample tube rack configured to hold a plurality of sample tubes.

Herein, the term "respective sample tube" indicates that each log file entry may refer to a corresponding sample tube. In general, different log file entries may refer to different sample tubes. Alternatively or in addition, the term "given sample tube" indicates that each log file entry may refer to the same sample tube, i.e. different log file entries may refer to the same sample tube.

In this embodiment, the method allows for parallelizing, e.g. multi-threading, by registering and assessing anomalies by reviewing events related to several tubes in the same log file. In particular, the log data which may be fed to a machine learning algorithm may refer to the same tube. Tube parallelization simplifies and/or improves the assessment of the presence of anomalies, as e.g. it allows for discerning tube anomalies from instrument anomalies and/or for detecting the problematic tubes.

In an embodiment, constructing the log data may comprise, for each log data item of the first plurality of log data items, assessing whether the respective log file entry refers to a given sample tube. For example, if the respective log file entry refers to the given sample tube, constructing the log data comprises selecting the respective log file entry.

According to this embodiment, when constructing the log data, the first computing device may assess whether the log file entries used to construct the log data items refer to the same sample tube.

In an embodiment, each log file entry of the first plurality of log file entries may comprise a fourth field, the fourth field comprising sample tube data, wherein the sample tube data may comprise information indicative of the respective sample tube, to which each log file entry refers. Additionally, for each log data item of the first plurality of log data items, assessing whether the respective log file entry refers to the given sample tube may be carried out by using the sample tube data comprised in the fourth field of the respective log file entry.

The fourth field may be specified similarly as described above for the first, second and third fields.

The sample tube data may comprise data identifying a patient and/or sample contained in the sample tube and/or the test and/or analysis to be carried out on the sample tube. For example, tube data comprises, e.g. consists of, an alphanumeric string that uniquely identifies the sample tube to which the log entry refers.

In this embodiment, each log file entry of the log file may comprise a tube field ("fourth field") with the barcode of the tube it refers to. Moreover, when constructing the log data, the first computing device may use the tube fields of the log file entries to assess whether the log file entries used to construct the log data items refer to the same tube.

In an embodiment, for at least a first log data item of the first plurality of log data items, the first field of the respective log file entry may specify that a sample tube has reached a location of the laboratory instrument and the second field of the respective log file entry may comprise information specifying the location which the sample tube has reached.

For example, the first field may consist of the string "SiteReached". If this is the case the second field may, depending on the location reached, consist of one of the strings "TrackConnection", "LeftCentrifuge", "AnalyserAU5800", "InputRegion", "OutputRegion", "Storage", "Decapper, "Recapper", and the like.

Additionally or alternatively, for at least a second log data item of the first plurality of log data items, the first field of the respective log file entry may specify that a sample tube has left a location of the laboratory instrument and the second field of the respective log file entry may comprise information specifying the location which the sample tube has left.

For example, the first field may consist of the string "SiteExited". If this is the case, the second field may, depending on the location that the sample tube has left, consist of one of the strings "TrackConnection", "LeftCentrifuge", "AnalyserAU5800", "InputRegion", "OutputRegion", "Storage", "Decapper, "Recapper", and the like.

Additionally or alternatively, for at least a third log data item of the first plurality of log data items, the first field of the respective log file entry may specify that a state of a sample tube has changed and the second field of the respective log file entry may comprise information specifying the current state of the sample tube.

For example, the first field may consist of the string "SampleStateChanged". If this is the case the second field may, depending on the current state of the sample tube, consist of one of the strings "ReadyForSchedulingState", "WaitForNewRoutePlanState", "RunningState", "CompletedState", "SampleAtTimeoutSiteState", and the like.

Additionally or alternatively, for at least a fourth log data item of the first plurality of log data items, the first field of the respective log file entry may specify that a task of the laboratory is started and the second field of the respective log file entry may comprise information specifying which task is started.

For example, the first field may consist of the string "InstructionStarted". If this is the case the second field may, depending on task started, consist of one of the strings "Decaplnstruction", "Recaplnstruction", "CentrifugeInstruction", "AnalyserAU5800Instruction", and the like.

Additionally or alternatively, for at least a fifth log data item of the first plurality of log data items, the first field of the respective log file entry may specify that a task of the laboratory is finished and the second field of the respective log file entry may comprise information specifying which task is finished.

For example, the first field may consist of the string "InstructionFinished". If this is the case the second field may, depending on task started, consist of one of the strings "Decaplnstruction", "Recaplnstruction", "Centrifugelnstruction", "AnalyserAU5800Instruction", and the like.

Additionally or alternatively, for at least a sixth log data item of the first plurality of log data items, the first field of the respective log file entry may specify that a route plan for a sample tube has been assessed and the second field of the respective log file entry may comprise information specifying whether the route plan has been accepted or rejected.

For example, the first field may consist of the string "ProcessRoutePlanAcceptance". If this is the case the second field may, depending on whether the route plan has been accepted or rejected, consist of the string "True" or "False".

In particular, the location of the laboratory may refer to a region and/or component of the laboratory device. For example, the location, i.e. the region or component of the laboratory device, may be an analyser, a pre-processing instrument, a post processing instrument or a track of the laboratory instrument..

The term "state" may refer to the status of a sample with regards to the testing, the processing and/or the analyzing of the sample, i.e. whether the sample is waiting for getting tested or a specific test has already been performed, for example.

According to the present invention a task may be the performing, by the laboratory instrument, of a processing step, e.g. a test step and/or analysis step for testing and/or analyzing a sample tube, respectively. For example, it may refer to the performing specific test, such as a PCR test and the like, and/or analysis such as, spectroscopic analysis and the like. For instance, a task may be the performing of a processing step such as the step of centrifuging, decapping, recapping, storing, and the like.

Generally, a route plan of a laboratory instrument, e.g. of an ALS, comprises information indicative of how to process the biological sample by that laboratory instrument, e.g. ALS. In other words, a route plan comprises one or more processing steps, e.g. a chronological sequence thereof.

In an embodiment, the method may further comprise the step of printing and/or displaying the log data and/or anomaly data to a user.

Thereby, the user may be provided with guidance as to how to resolve the anomaly detected by the method according to the first aspect of the invention or any of the corresponding embodiments.

In an embodiment, the constructing the log data may comprise, for each log data item of the first plurality of log data items,
∘ selecting fifth field-data of a fifth field of the respective log file entry, the fifth field of the respective log file entry further describing the respective event which has occurred in the laboratory instrument, wherein the selecting of the fifth field is based on the first field-data of the respective first field and/or the second field-data of the respective second field of the respective log file entry; and
wherein the constructing of said each log data item is carried out by using the fifth field-data of the fifth field of the respective log file entry.

In particular, for at least a log data item of the first plurality of the log data items the selecting of the fifth field-data of the fifth field of the respective log file entry comprises determining a respective position in the respective log file entry by using the first field data and/or the second field data, choosing as fifth field the field at the respective position in the respective log file entry and accessing the data contained in the fifth field, the latter data being the fifth field data.

The fifth field data may for instance comprise, e.g. consist of, an alphanumeric string. For example if the first field data consists of the string "SiteReached", and the second field data is the string "Recapper", the fifth field may be the string "RecapStop". For example, in this case, the resulting log data item may be constructed by concatenating the aforementioned strings separated by a dot, e.g. the log file data item may consist of the string "SiteReached.Recapper.RecapStop".

In particular, in some embodiments, for at least the first log data item of the first plurality of log data items, the first field of the respective log file entry specifies that a sample tube has reached a location of the laboratory instrument and the second field of the respective log file entry comprises information specifying the location which the sample tube has reached. In said embodiments, for at least the second log data item of the first plurality of log data items, the fifth field of the respective log file entry specifies the state of the task carried out at the location specified in the second field. For example, if the first field and the second field consist of the string "SiteReached" and "Decapper", respectively, the fifth field may, depending on the state of the centrifugation task, consist of one of the strings, "Decap", "DecapStop", and the like.

A second aspect of the present invention relates to a computer-implemented method comprising the steps of:
- providing a log file of a laboratory instrument to a computing device, the log file comprising a first plurality of log file entries, each log file entry of the first plurality of log file entries comprising a plurality of fields;
- obtaining anomaly data, the anomaly data comprising information indicative of the presence of an anomaly in the laboratory instrument,
wherein the computing device is configured to construct log data, the log data comprising a first plurality of log data items, by, for each log data item of the first plurality of log data items:
o selecting first field-data of a first field of a respective log file entry, the first field of the respective log file entry specifying a respective event which has occurred in the laboratory instrument;
∘ selecting second field-data of a second field of the respective log file entry, the second field of the respective log file entry further describing the respective event which has occurred in the laboratory instrument, wherein the selecting of the respective second field is based on the first field-data of the respective first field of the respective log file entry; and
∘ constructing said each log data item by using the first field-data of the first field of the respective log file entry and the second field-data of the second field of the respective log file entry,
and wherein the computing device is configured to assess, by using the log data, for the presence of an anomaly in the laboratory instrument.

All advantages, specifications, explanations, additional features and embodiments of the previous aspect also apply to the second aspect of the invention. In particular, the method according to the second aspect of the invention allows for a better, faster and/or easier processing of data and/or for a better, faster and/or easier detection of anomalies. Hence, the reliability of the results of sample analyses provided by, for example, laboratory instruments of an automated laboratory system is increased.

In particular, the method according to the first aspect of the present invention may be carried out by a computing device comprised in the laboratory instrument. According to the second aspect, for example, the computing device of the laboratory instrument provides the log file to a server that carries out the method according to the first aspect of the present invention. Additionally or alternatively, any of the features of the second computing device according to the first aspect of the presence invention may apply to the computing device according to the second aspect of the present invention.In some embodiments, the step of obtaining anomaly data, may comprise accessing said data. In particular, said data may be created and/or provided by the computing device.

All methods according to the first aspect and/or second aspect of the invention may be performed on the fly while a sample is being processed in a laboratory comprising one or more laboratory instruments.

A third aspect of the present invention relates to a computer-implemented method comprising the steps of:
- accessing a log file of a laboratory instrument, the log file comprising a first plurality of log file entries, each log file entry of the first plurality of log file entries comprising a plurality of fields;
- constructing labelled training log data, the labelled training log data comprising a plurality of training log data items and label data; and
- initiating the training of a machine learning algorithm, wherein the training of the machine learning algorithm is carried out by using the labelled training log data,
wherein the step of constructing the labelled training log data comprises, for each training log data item of the plurality of training log data items,
∘ selecting first field-data of a first field of a respective log file entry, the first field of the respective log file entry specifying a respective event which has occurred in the laboratory instrument;
∘ selecting second field-data of a second field of the respective log file entry, the second field of the respective log file entry further describing the respective event which has occurred in the laboratory instrument, wherein the selecting of the second field is based on the first field-data of the first field of the respective log file entry; and
o constructing said each training log data item by using the first field-data of the first field of the respective log file entry and the second field-data of the second field of the respective log file entry.

For example, the additional features referring to the log data items according to the first aspect of the invention may also apply to the training log data items according to the third aspect of the invention. In particular, the additional features referring to the constructing of the log data items according to the first aspect of the invention may also apply to the constructing of the training log data items according to the third aspect of the invention.

The label data may comprise information indicative of the ground truth associated to the plurality of training log data items. In particular, the ground truth specifies the desired output that the training machine learning algorithm is required to generate by processing the plurality of log data items. For example, the label data comprise information indicative of one or more expected benchmark log data items. In particular, any of the various features of the one or more expected benchmark data items of the first aspect of the invention can be included or combined with the features of the one or more expected benchmark log data items of the third aspect of the invention.

For example, the label data may comprise, for each expected benchmark log data item of the one or more expected benchmark log data items, the probability that, in absence of anomalies, the respective event associated to said each expected benchmark log data item immediately follows the events related to the training log data items. In this case, each expected benchmark log data item of the one or more expected benchmark log data items is one of the log data items that, in absence of anomalies, are expected to immediately follow the training log data items. In some embodiments, the label data specifies a log data item that immediately follow the training log data items. For instance, the label data may consist of the log data item that immediately follow the training log data items.

In particular, the information comprised in the label data can be the same as the information comprised in the output data of the first aspect of the present invention. For example, during the training, the information comprised in the label data is compared with the information comprised in the output of the machine learning algorithm. Typically, the training is carried out by using a plurality of labelled log data.

In some embodiments, a plurality of labelled training log data is constructed by obtaining a non-anomalous log file and by grouping the log file entries into a plurality of training groups containing N log file entries each. In particular, for each training group, the N log file entries of said group consist of a first log file entry, which refers to a respective sample tube or to a respective laboratory instrument component, and the first N-1 log file entries that follow the first log file entry in the log file and refer to said respective sample tube or said respective laboratory instrument component. In particular, each labelled training log data of the plurality of labelled training log data is obtained by a respective training group of the plurality of training groups. In particular, the training log data items constructed from the first N-1 log file entries of the respective training group. The expected benchmark log data item, which constitutes the label data, is constructed by using the N^{th} entry of the respective training group. The plurality of labelled training log data is then used to train the machine learning algorithm.

All advantages, specifications, explanations, additional features and embodiments of the previous aspects also apply to the third aspect of the invention.

The initiating of the training of the machine learning algorithm may comprise instructing a computing device to do some action that starts the training, the computing device being for example a client device and/or server. The initiating may comprise providing training data to the computing device.

In an embodiment of the third aspect, the step of initiating the training of a machine learning algorithm may be carried out by providing the labelled training log data to a fourth computing device, the fourth computing device being configured to train the machine learning algorithm by using the labelled training log data.

In particular, the fourth computing device may be the second computing device described herein.

In an alternative embodiment, the step of initiating the training of a machine learning algorithm may be carried out by training the machine learning algorithm by using the labelled training log data.

All methods according to the first aspect and/or second aspect and/or third aspect of the invention may be performed on the fly while a sample is being processed in a laboratory comprising one or more laboratory instruments.

A fourth aspect of the present invention relates to a data processing system comprising a processor configured to carry out the method according to one of the preceding aspects or corresponding embodiments.

The system may comprise at least one of a first computing device, a second computing device, a third computing device or a fourth computing device. In particular, at least two of the second computing device, the third computing device and the fourth computing device may be the same. All computing devices may be different from each other. The computing devices may in particular be located in different laboratories or laboratory sites. In particular, the first computing device may be comprised by or be a part of the laboratory instrument.

A fifth aspect of the present invention relates to a laboratory instrument comprising the data processing system according to the fourth aspect.

A sixth aspect of the present invention relates to an automated laboratory system comprising the data processing system according to the fifth aspect.

A seventh aspect of the present invention relates to a computer program product comprising instructions which, when the program is executed by a computer system, cause the computer system to carry out the method according to the first aspect or the second aspect or the third aspect or any of the corresponding embodiments.

An eighth aspect of the present invention relates to a computer-readable medium comprising instructions which, when executed by a computer system, cause the computer system to carry out the method according to the first aspect or the second aspect or the third aspect or any of the corresponding embodiments.

All advantages, specifications, explanations, additional features and embodiments of the first aspect, second aspect and third aspect also apply to the fourth aspect, the fifth aspect, the sixth aspect, the seventh aspect, and the eighth aspect of the invention.

### Description of the figures

Hereinbelow, the figures are described. The figures depict one or more embodiments of the present invention. Individual features depicted in the figures may be combined to further embodiments. The figures show:
- **Fig. 1:**: a schematic representation of an exemplary first computing device and of an exemplary second computing device according to an aspect of the invention;
- **Fig. 2:**: a first swim lane diagram including exemplary steps performed by the first computing device and the second computing device;
- **Fig. 3:**: a second swim lane diagram including exemplary steps performed by the first computing device and the second computing device;
- **Fig. 4:**: a schematic representation of an excerpt of an exemplary log file; and
- **Fig. 5:**: a schematic representation of an excerpt of exemplary log data.

### Detailed Description

In the following, method steps denoted with the same reference number are carried out in the same way.

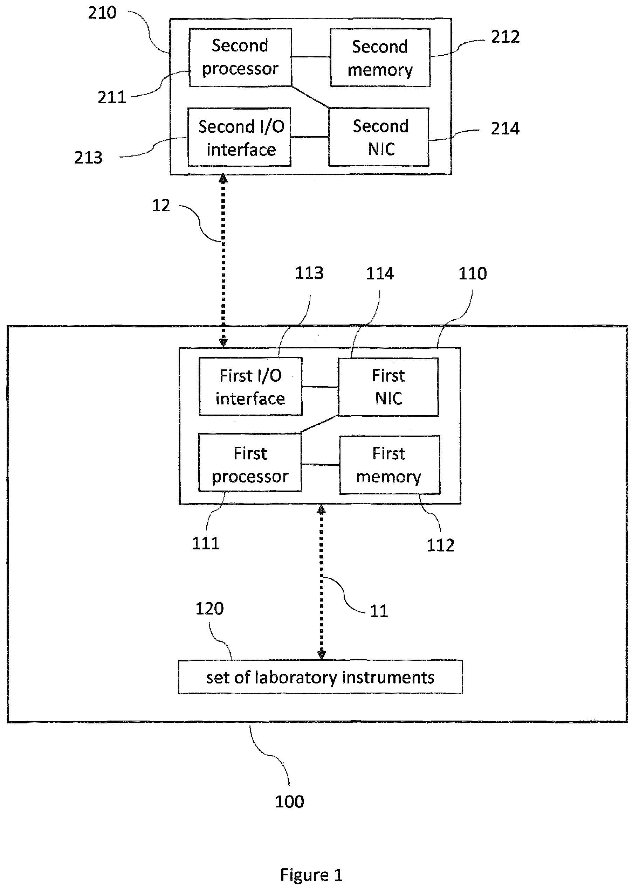

**Figure 1** (Fig. 1) shows a schematic representation of an exemplary first computing device 110 and of an exemplary second computing device 120 according to an aspect of the present invention.

In the embodiment shown in figure 1, the first computing device 110 is a cluster computer located at a laboratory 100 which comprises a set of laboratory instruments 120 for processing biological samples. The first computing device 110 may also be a desktop PC, a server, a distributed computing system or a cloud computing system.

The first computing device 110 comprises a first processor 111, a first memory 112 or storage unit, a first I/O interface 113 and a first interface controller 114 (NIC). The first processor 111 is connected to the first memory 112 and the first NIC 114. The first NIC 114 is connected to the first I/O interface 113. The first I/O interface 113 is configured to send output of the first computing device 110 to or receive input from at least one second computing device 210 by means of a protocol suite to exchange data 12. In figure 1, only one second computing device 210 is shown.

The second computing device 210 shown in figure 1 is located at a location different from the location of the laboratory 100. The second computing device 210 is located at the laboratory 100. The second computing device 210 may be a desktop PC, a workstation, a cluster computer or a server. The second computing device 210 may be comprised by or be a computing unit of one or more of the laboratory instruments 220 present in the laboratory 100. The second computing device 210 comprises a second processor 211, a second memory 212 or storage unit, a second I/O interface 213 and a second NIC 214. The second NIC 214 is connected to the second processor 211 and the second I/O interface 213. The second processor 211 is also connected to the second memory 212.

The first computing device 110 may output instructions to or receive data from a set of laboratory instruments 120 which are located at the location of the laboratory 100. The input and output are transmitted between the first computing device 110 and the set of laboratory instruments 120 by means of the protocol suite to exchange data 11. In some embodiments, the first computing device 110 may be comprised in an automated laboratory system (not shown) that comprises the set of laboratory instruments 120.

Via the protocol suite 12, the first computing device 110 may transmit data and/or instructions to the second computing device 210 and vice-versa. The first computing device 110 may receive the instructions and initiate the performing of one or more clinical tests on a sample by instructing one or more of the set of laboratory instruments 120 at the laboratory 100. The set of lab instruments 120 may for instance be comprised in an automated laboratory system that comprises or is in data communication with the first computing device 110.

The first and second computing devices 110, 210 may comprise more than one processor 111, 211 for task parallelization. The first memory 112 as well as the second memory 212 may comprise or be a RAM and/or a magnetic hard drive and/or a solidstate storage and/or a cloud storage.

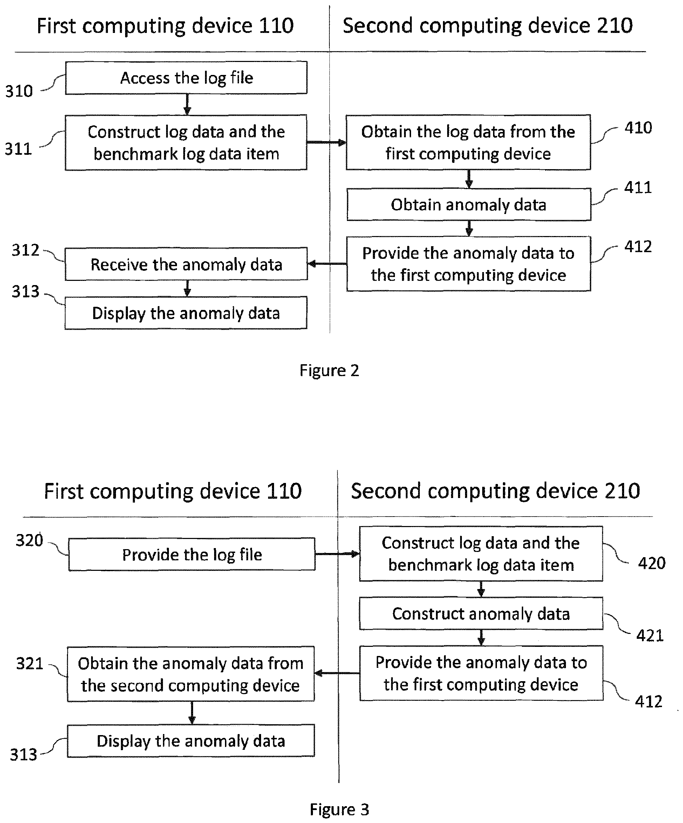

**Figure 2** (Fig. 2) shows a first swim lane diagram including exemplary steps performed by the first computing device and the second computing device. In this embodiment, the second computing device 210 carries out an embodiment of the method according to the first aspect of the invention. In particular, the second memory 212 stores a computer program comprising instructions which, when executed by the processor 211, cause the second computing device 210 to carry out the method according to the first aspect of the present invention.

In a step 310, in particular a first step, the first computing device 110 as shown in Fig. 1, accesses a log file of the set of laboratory instruments 120. In particular, the first computing device may generate the log file by using log information provided by the set of laboratory instruments 120.

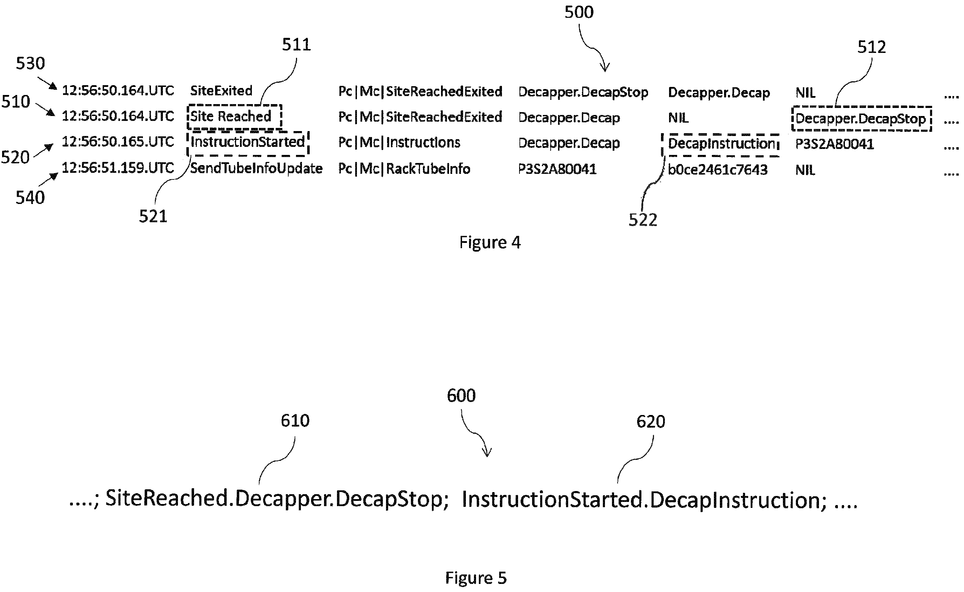

**Figure. 4** (Fig. 4) shows a schematic representation of an excerpt 500 of an exemplary log file. In particular, the log file is a text file and comprises a plurality of log file entries 510, 520, 530, 540, each log file entry being a line of the log file. The log file entries 510, 520, 530, 540 are arranged in the log file chronologically according to the creation time of the log file entries, e.g. from the least recently generated entry to the most-recently generated entry. In particular, the entries of the log files may be arranged according to their timestamps. In some cases, e.g. if log file entries are generated by different instruments of the set of laboratory instruments 120, two or more log file entries may have the same timestamp. In particular, in this case, those two or more log file entries are arranged randomly with respect to one another and chronologically with respect to the other log file entries.

Each entry of the log file entries consists of a plurality of fields 511, 512, 521, 522, each field of the log entries 510, 520, 530, 540 consisting of an alphanumeric string that comprises the respective field-data. The fields of each log entry are separated from one another by one or more blank spaces. For example, the log entry 530 comprises the fields "12:56:50.164.UTC", "SiteExited", "Pc|Mc|SiteReachedExited", "Decapper.DecapStop", and "Decapper.Decap".

With reference to Fig. 2, at step 311, the first computing device 110 constructs the log data and the benchmark log data item, L_{N}. The log data which comprises a first plurality of log data items, L₁, L₂, ..., L_{N-1}. **Figure 5** (Fig. 5) shows a schematic representation of an excerpt of exemplary log data 600. In particular, the log data are collected in a text file comprising a plurality of log file entries 610, 620, each log data item consisting of an alphanumeric string. The log data items 610, 620 are arranged according to the order of the respective log entries 610, 620. In particular, the constructing of the log data comprises, for each log data items 610, 620 of the first plurality of log data items:
o selecting first field-data of a first field 511, 521 of a respective log file entry 510, 520, the first field 511, 521 of the respective log file entry 510, 520 specifying a respective event which has occurred in the set of laboratory instruments 120;
o selecting second field-data of a second field 512, 522 of the respective log file entry 510, 520, the second field 512, 522 of the respective log file entry 510, 520 further describing the respective event which occurred in the set of laboratory instruments 120; and
o constructing said each log data item 610, 620 by using the first field-data of the first field 511, 521 of the respective log file entry 510, 520 and the second field-data of the second field 512, 522 of the respective log file entry 510, 520.

According to the present embodiment, for each log data item 610, 620 of the first plurality of the log data items, the selecting of the first field-data of the first field 511, 521 of the respective log file entry 510, 520 consists of choosing as first field the field at the second position in the log file entry, said field specifying an event which has occurred at or in the set of laboratory instruments 120. For example, the field at the second position of the log entry 510 is the string "Site Reached" 511, which specifies that a sample tube has reached a location of the set of laboratory instruments 120. The field at the second position of the log entry 520 is the string "InstructionStarted" 521, which specifies that a task of the laboratory is started.

According to the present embodiment, for each log data item 610, 620 of the first plurality of the log data items, the selecting of the second field-data of the second field 512, 522 of the respective log file entry 510, 520 is carried determining a respective position in the respective log file entry 510, 520 by using the first field data, choosing as second field 512, 522 the field at the respective position in the respective log file entry 510, 520.

The respective position may be determined by using a first look-up table (not shown) correlating the possible values of the first field data with the positions on the log file entries. For instance, according to the first look-up table, if the first field-data is the string "Site Reached" 511, the respective position in the log file entry is the sixth one which, in the log file entry 510, is the string "Decapper.DecapStop", which specifies that the sample has reached the decapper. In particular, the latter string specifies the location which the sample tube has reached. Moreover, for instance, according to the first look-up table, if the first field-data is the string "InstructionStarted" 521, the respective position in the log file entry is the fifth one which, in the log file entry 520 is the string "Decaplnstruction". In particular, the latter string specifies that a task started, in this case the processing step of decapping.

Alternatively, in some embodiments, if the log file is organized by a using mark-up language, the selecting of the second entry may be carried out by selecting, based on the first entry, the appropriate tag among the tags which marks the entries of the log file entry.

Each log data item 610, 620 of the first plurality of log data items are constructed by concatenating the first field 511, 521 and the second field 512, 522 of the respective log file entry 510, 520 with a separator, in this case a dot. For example, the log data item "SiteReached.Decapper.DecapStop" 610 is constructed by concatenating the string "SiteReached" 511 with the string "Decapper.DecapStop" 512. Similarly, the log data item "InstructionStarted.DecapInstruction" 620 is constructed by concatenating the string "InstructionStarted" 521 with the string "Decaplnstruction" 522.

The construction of the benchmark log data item is carried out as the construction of the log data items 610, 620 described above.

With reference to Fig. 2, In a next step 410, the second computing device 220 obtains the log data from the first computing device 110 by receiving said data which are sent by the first computing device 110. Alternatively, the second computing device 220 obtains the log data from the first computing device 110 by accessing the log data at a memory device, that stores the log data, the log data being made available to the second computing device 210 by the first computing device 110.

In a next step 411, the second computing device 210 obtains anomaly data. In this embodiment, obtaining anomaly data comprises assessing for the presence of an anomaly by using the log data items L₁, L₂, ..., L_{N-1} and the benchmark log data item L_{N}. In particular, the log data items L₁, L₂, ..., L_{N-1} are used as input of a trained machine learning algorithm, e.g. a trained long short-term memory recurrent neural network. The trained machine learning algorithm generates the output data. The output data comprise information specifying, for each lemma of the log data vocabulary, the probability that, in absence of an anomaly, said each lemma would immediately follow the log data items L₁, L₂, ..., L_{N-1}. In this case, the presence of the anomaly is assessed by determining whether the probability associated to L_{N} is greater than or equal to a given threshold, e.g. 0.05. In particular, an anomaly is detected if this probability is lower than said threshold and, if an anomaly is detected, anomaly data are generated. In this case, in particular, an anomaly is detected and hence, the anomaly data are generated. The anomaly data specify that an anomaly has been detected. Additionally, the anomaly data may comprise the resolution data comprising information indicative of one or more actions for resolving the anomaly present in the set of laboratory instruments 120.

At step 412, the second computing device 412 provides the anomaly data to the first computing device by sending said data to the first computing device 110. The second computing device 210 may provide, transmit, send or output the anomaly data over a network, in particular the protocol suite 12, to the first computing device 110.

At step 312, the first computing device 110 receives the anomaly data from the second computing device 210. At step 313, the first computing device 110 provides the anomaly data to a user e.g. by displaying said data on a screen. In particular, the first computing device 110 may provide one or more actions for resolving the detected anomaly. Specifically, the first computing device 110 may output and/or print and/or display the one or more actions to resolve the detected anomaly to the user.

For example, the anomaly detected may relate to incomplete tests which are performed on a sample, such as incomplete centrifugation and/or incomplete growth of biomaterial, and/or downtimes of one or more laboratory instruments and/or unavailabilities of one or more of the laboratory instruments. The first computing device 110 may provide suggestions as to how to resolve these anomalies to a user. For example, the first computing device 110 may instruct a user to check and/or investigate whether the centrifuge works properly. The first computing device 110 may also print and/or display error messages and/or notifications to the user, wherein the error messages and/or notifications are related to certain anomalies which may be detectable by the machine learning algorithm.

In the case in which the first and first computing device 110 is comprised in an automated laboratory system, the second computing device 210 may also detect an anomaly in a laboratory instrument of the set of laboratory instruments 120 and instruct the first computing device 110 to resolve the anomaly detected based on the anomaly and resolution data. For example, the resolution of an anomaly may be done by rerouting one or more sample tubes to bypass the one or more instruments of the set of the laboratory instruments 120 in which an anomaly was detected. Thereby, the automated laboratory system may be able to check for and correct an anomaly autonomously.

In this case, the first computing device 110 carries out an embodiment of the method according to the first aspect of the present invention. In particular, in this embodiment the step of obtaining the log data is carried out by the first computing device 110, by constructing said log data (step 311 described above) and the step of obtaining anomaly data is carried out by receiving the anomaly data from the second computing device 210 (step 312 as described above).

**Figure 3** (Fig. 3) shows a second swim lane diagram including exemplary steps performed by the first computing device and the second computing device and the third computing device. In this embodiment, the first computing device 110 carries out an embodiment of the method according to the second aspect of the invention. In particular, the first memory 112 stores a computer program comprising instructions which, when executed by the processor 111, cause the first computing device 110 to carry out the method according to the second aspect of the present invention.

At step 320, the first computing device 110 provides, e.g. sends, the log file to the second computing device 210.

At step 420, the second computing device construct log data and the benchmark log data item. The construction of the log data and the benchmark log data item is carried out according to the step 311 of Fig. 2 described above. At step 421, the second computing device 210 constructs the anomaly data. The construction of the anomaly data and the benchmark log data item is carried out according to the step 411 of Fig. 2 described above. At step 412, the second computing device 210 provides, e.g. sends, the anomaly data to the first computing device 110.

At step 321, the first computing device 110 obtains the anomaly data from the second computing device 210 and, at step 313 displays the anomaly data to the user. The anomaly data are obtained by the first computing device 110 by receiving them from the second computing device 210, according to the step 312 of Fig. 2 described above.

In this case, the second computing device 210 carries out an embodiment of the method according to the first aspect of the present invention. In particular, in this embodiment the step of obtaining the log data is carried out by the second computing device 110, by constructing said log data (step 420 described above) and the step of obtaining anomaly data is carried out by constructing the anomaly data (step 421 as described above).

## Claims

1. A computer-implemented method comprising the steps of:
- obtaining (410), from or by a first computing device (110), log data (600), the log data comprising a first plurality of log data items (610, 620) ; and
- obtaining (411) anomaly data,
wherein the first computing device (110) is configured to access (310) a log file of the laboratory instrument (120), the log file (500) comprising a first plurality of log file entries (510, 520, 530, 540), each log file entry of the first plurality of log file entries comprising a plurality of fields (511, 512, 521, 522), and to construct (311) the log data (600), by, for each log data item (610, 620) of the first plurality of log data items:
o selecting first field-data of a first field (511, 521) of a respective log file entry (510, 520), the first field (511, 521) of the respective log file entry (510, 520) specifying a respective event which has occurred in the laboratory instrument (120);
o selecting second field-data of a second field (512, 522) of the respective log file entry (510, 520), the second field (512, 522) of the respective log file entry (510, 520) further describing the respective event which has occurred in the laboratory instrument (120), wherein the selecting of the respective second field is based on the first field-data of the respective first field of the respective log file entry; and
o constructing said each log data item by using the first field-data of the first field of the respective log file entry and the second field-data of the second field of the respective log file entry;
wherein the anomaly data comprises information indicative of the presence of an anomaly in a laboratory instrument (120) and the presence of the anomaly is assessed by using the log data (600).

2. The method according to claim 1, wherein the step of obtaining the anomaly data comprises assessing, by using the log data, for the presence of the anomaly in the laboratory instrument (120) and generating the anomaly data.

3. The method according to claim 2, wherein the log data are obtained from the first computing device (110) and the step of obtaining the anomaly data s carried out by a second computing device (210), the second computing device (210) being different from the first computing device (110).

4. The method according to either claim 2 or claim 3, wherein the assessing for the presence of an anomaly in the laboratory instrument (120) is carried out by using the log data as input to at least one of a machine learning algorithm and/or a decision tree.

5. The method according to any one of the claims 2 to 4, further comprising the step of:
- obtaining, by or from the first computing device (110), a benchmark log data item by using a respective benchmark log file entry of the log file,
wherein the assessing for the presence of an anomaly in the laboratory instrument is based on the benchmark log data item.

6. The method according to any one of the claims 2 to 5, wherein, if it has been assessed that the anomaly is present in the laboratory instrument (120), the method further comprises the step of:
- obtaining resolution data, the resolution data comprising information indicative of one or more actions for resolving the anomaly present in the laboratory instrument (120).

7. The method according to any one of claims 2 to 6, wherein the step of obtaining, by the first computing device (110), log data comprises:
- accessing the log file of the laboratory instrument (120); and
constructing the log data, wherein the step of constructing the log data comprises, for each log data item of the first plurality of log data items:
o selecting the first field-data of the first field of the respective log file entry;
o selecting the second field-data of the second field of the respective log file entry, wherein the selecting of the second field is based on the first field-data of the first field of the respective log file entry; and
o constructing said each log data item by using the first field-data of the first field of the respective log file entry and the second field-data of the second field of the respective log file entry.

8. The method according to claim 1, further comprising the step of:
- providing, by the first computing device (110), the log data to a third computing device, the third computing device being configured to assess, by using the log data, for the presence of the anomaly in the laboratory instrument (120),
and wherein the step of obtaining, by the first computing device (110), log data comprises:
- accessing the log file of the laboratory instrument (120); and
- constructing the log data, wherein the step of constructing the log data comprises, for each log data item of the first plurality of log data items:
o selecting the first field-data of the first field of the respective log file entry;
o selecting the second field-data of the second field of the respective log file entry, wherein the selecting of the second field is based on the first field-data of the first field of the respective log file entry; and
o constructing said each log data item by using the first field-data of the first field of the respective log file entry and the second field-data of the second field of the respective log file entry.

9. The method according to any one of the previous claims, wherein each log file entry of the first plurality of log file entries refers to a respective sample tube, and/or wherein, for each log data item of the first plurality of log data items, the respective log file entry refers to a given sample tube.

10. The method according to any one of the previous claims, wherein:
i. for at least a first log data item of the first plurality of log data items, the first field of the respective log file entry specifies that a sample tube has reached a location of the laboratory instrument (120) and the second field of the respective log file entry comprises information specifying the location which the sample tube has reached, and/or
ii. for at least a second log data item of the first plurality of log data items, the first field of the respective log file entry specifies that a sample tube has left a location of the laboratory instrument (120) and the second field of the respective log file entry comprises information specifying the location which the sample tube has left, and/or
iii. for at least a third log data item of the first plurality of log data items, the first field of the respective log file entry specifies that a state of a sample tube has changed and the second field of the respective log file entry comprises information specifying the current state of the sample tube, and/or
iv. for at least a fourth log data item of the first plurality of log data items, the first field of the respective log file entry specifies that a task of the laboratory is started and the second field of the respective log file entry comprises information specifying which task is started, and/or
v. for at least a fifth log data item of the first plurality of log data items, the first field of the respective log file entry specifies that a task of the laboratory is finished and the second field of the respective log file entry comprises information specifying which task is finished, and/or
vi. for at least a sixth log data item of the first plurality of log data items, the first field of the respective log file entry specifies that a route plan for a sample tube has been assessed and the second field of the respective log file entry comprises information specifying whether the route plan has been accepted or rejected.

11. A computer-implemented method comprising the steps of:
- providing (320) a log file (500) of a laboratory instrument (120) to a computing device ( 210), the log file (500) comprising a first plurality of log file entries (510, 520, 530, 540), each log file entry of the first plurality of log file entries comprising a plurality of fields;
- obtaining (321) anomaly data, the anomaly data comprising information indicative of the presence of an anomaly in the laboratory instrument (120),
wherein the computing device (210) is configured to construct log data (600), the log data comprising a first plurality of log data items (610, 620), by, for each log data item of the first plurality of log data items:
o selecting first field-data of a first field (511, 521) of a respective log file entry (510, 520), the first field of the respective log file entry specifying a respective event which has occurred in the laboratory instrument (120);
o selecting second field-data of a second field (512, 522) of the respective log file entry (510, 520), the second field of the respective log file entry further describing the respective event which has occurred in the laboratory instrument (120), wherein the selecting of the respective second field is based on the first field-data of the respective first field of the respective log file entry; and
o constructing said each log data item by using the first field-data of the first field of the respective log file entry and the second field-data of the second field of the respective log file entry,
and wherein the computing device (210) is configured to assess, by using the log data, for the presence of an anomaly in the laboratory instrument (120).

12. A computer-implemented method comprising the steps of:
- accessing a log file of a laboratory instrument, the log file comprising a first plurality of log file entries, each log file entry of the first plurality of log file entries comprising a plurality of fields;
- constructing labelled training log data, the labelled training log data comprising a plurality of training log data items and label data; and
- initiating the training of a machine learning algorithm, wherein the training of the machine learning algorithm is carried out by using the labelled training log data, wherein the step of constructing the labelled training log data comprises, for each training log data item of the plurality of training log data items,
o selecting first field-data of a first field of a respective log file entry, the first field of the respective log file entry specifying a respective event which has occurred in the laboratory instrument (120);
o selecting second field-data of a second field of the respective log file entry, the second field of the respective log file entry further describing the respective event which has occurred in the laboratory instrument (120), wherein the selecting of the second field is based on the first field-data of the first field of the respective log file entry; and
o constructing said each training log data item by using the first field-data of the first field of the respective log file entry and the second field-data of the second field of the respective log file entry.

13. A data processing system comprising a processor configured to carry out the method according to any one of the preceding claims.

14. A computer program product comprising instructions which, when the program is executed by a computer system, cause the computer system to carry out the method according to any one of the claims 1 to 12.

15. A computer-readable medium comprising instructions which, when executed by a computer system, cause the computer system to carry out the method according to any one of the claims 1 to 12.
